# EUROPEAN PATENT SPECIFICATION

(11) **EP 2 624 821 B1**
(45) Date of publication and mention of the grant of the patent: **27.06.2018**
(21) Application number: 11830119.1
(22) Date of filing: 05.10.2011
(51) Int. Cl.: A61K 31/10, A61K 31/42, A61P 25/22, A61K 31/137, A61K 31/7008, A61P 25/24, A61K 31/138, A61K 31/714, A61P 25/28, A61K 31/198, A61P 25/00, A61P 43/00

(54) **COMBINATION THERAPY FOR THE TREATMENT OF DEPRESSION AND OTHER NON-INFECTIOUS DISEASES**
KOMBINATIONSTHERAPIE ZUR BEHANDLUNG VON DEPRESSIONEN UND ANDEREN NICHTINFEKTIÖSEN ERKRANKUNGEN
POLYTHÉRAPIE DESTINÉE AU TRAITEMENT DE LA DÉPRESSION ET D'AUTRES MALADIES NON INFECTIEUSES

(30) Priority: 07.10.2010 AU 2010904479
(43) Date of publication of application: 14.08.2013
(73) Proprietor: Eaglepharma Pty Ltd, Mountain Creek, Queensland 4557 (AU)
(72) Inventor: TURNER, Paul, Mountain Creek Queensland 4557 (AU)
(74) Representative: Bridle, Andrew Barry
(86) International application number: PCT/AU2011/001275
(87) International publication number: WO 2012/045118

(56) References cited:
- US-A1- 2009 110 674
- US-A1- 2009 110 674

## Description

### FIELD OF THE INVENTION

THIS INVENTION relates to control of inflammation to treat or prevent non-infectious diseases, particularly psychiatric disorders. More particularly, this invention relates to methods, uses and compositions of a nutritional supplement that includes a therapeutically effective amount of methylsulfonylmethane, glucosamine, L-glycine, and vitamin B₁₂ (or a derivative of any one thereof) for treating or preventing non-infectious diseases, particularly psychiatric disorders.

### BACKGROUND OF THE INVENTION

The pro-inflammatory cytokines tumor necrosis factor-alpha (TNF-α), interleukin-1 (IL-1) and interleukin-6 (IL-6) are key players in the inflammatory response. The body has a system for turning on inflammation, the so called pro-inflammatory response, and turning off inflammation when its work is done, the anti-inflammatory response. The cytokines TNF, IL-1 and IL-6 are some of the major pro-inflammatory cytokines. The inflammatory response can, and does, cause significant disease presentations if the anti-inflammatory response is not effective in stopping the inflammation. Recent evidence has implicated the pro-inflammatory cytokines as being responsible for an increasing number non-infectious disease presentations throughout the world. Non-infectious diseases such as, for example, rheumatoid arthritis, cerebral palsy, Bell's palsy, major depression, Crohn's disease, anxiety, suicidal ideation, bipolar diseases, addictive behaviour, premature ageing, heart disease, Parkinson's disease, diabetes, attention deficit hyperactivity disorder (ADHD), autism, Alzheimer's disease, and others have all been implicated as being as a result of this process. Mounting data indicate that exposure to stress, a well-known precipitant of mood disorders, can also activate inflammatory responses both in the periphery and in the brain.

Although the evidence for the role of the pro-inflammatory response in disease processes is strong, this has not translated into therapeutic regimes. The small numbers of treatments that have been used to reduce the level of the pro-inflammatory response involve the production and use of monoclonal antibodies against TNF or IL-6. Results have been encouraging, but the treatment process itself is invasive, requiring the antibody to be injected, and significant adverse effects have been identified that make its general use for non-life-threatening diseases not a serious option. Thus, there is a need for effective, non-invasive therapies that can prevent, slow, stop, and/or reverse the pro-inflammatory response, particularly therapies that have few negative side-effects.

### SUMMARY OF THE INVENTION

The present invention is broadly directed to methods and compositions of a nutritional supplement that includes a therapeutically effective amount of methylsulfonylmethane, glucosamine, L-glycine, and vitamin B₁₂ (or a derivative of any one thereof) for treating or preventing non-infectious diseases resulting from the pro-inflammatory response, particularly psychiatric disorders. The therapeutic combination of methylsulfonylmethane, glucosamine, L-glycine, and vitamin B₁₂ is also known as GMGB1.

In a first aspect, the invention provides a method for treating a non-infectious disease in a subject in need thereof, the method including the step of administering to the subject a therapeutically effective amount of a pharmaceutical composition including (a) methylsulfonylmethane or a derivative thereof, (b) glucosamine or a derivative thereof, (c) L-glycine or a derivative thereof, and (d) vitamin B₁₂ or a derivative thereof.

In one embodiment, the invention provides a method for treating a mood disorder, an anxiety disorder, an attention deficit disorder, dementia, or stress in a subject in need thereof, the method including the step of administering to the subject a therapeutically effective amount of a pharmaceutical composition including (a) methylsulfonylmethane or a derivative thereof, (b) glucosamine or a derivative thereof, (c) L-glycine or a derivative thereof, and (d) vitamin B₁₂ or a derivative thereof.

Suitably, according to the aforementioned aspect, the pharmaceutical composition includes methylsulfonylmethane, glucosamine, L-glycine, and vitamin B₁₂.

Suitably, according to the aforementioned embodiment, treating a mood disorder, an anxiety disorder, an attention deficit disorder, dementia, or stress in a subject in need thereof includes reducing the severity of the mood disorder, the anxiety disorder, the attention deficit disorder, dementia, or stress in the subject.

In some embodiments of the aforementioned aspect, the method further includes administering to the subject one or more additional active agents, for example, a norepinephrine reuptake inhibitor, a selective serotonin reuptake inhibitor, a tricyclic antidepressant, and/or a monoamine oxidase inhibitor.

In a second aspect, the invention provides a method for improving cognitive performance in a subject, the method including the step of administering to the subject a therapeutically effective amount of a pharmaceutical composition including (a) methylsulfonylmethane or a derivative thereof, (b) glucosamine or a derivative thereof, (c) L-glycine or a derivative thereof, and (d) vitamin B₁₂ or a derivative thereof.

Suitably, according to the aforementioned aspect, the pharmaceutical composition includes methylsulfonylmethane, glucosamine, L-glycine, and vitamin B₁₂.

Suitably, according to the aforementioned aspects, the subject is a human.

In a third aspect, the invention provides use of methylsulfonylmethane or a derivative thereof, glucosamine or a derivative thereof, L-glycine or a derivative thereof, and vitamin B₁₂ or a derivative thereof in the manufacture of a medicament for treating a non-infectious disease in a subject.

Suitably, according to the aforementioned aspect, methylsulfonylmethane, glucosamine, L-glycine, and vitamin B₁₂ are used.

Suitably, according to the aforementioned aspect, the subject is a human.

In a fourth aspect, the invention provides a pharmaceutical composition for treating a non-infectious disease, including methylsulfonylmethane or a derivative thereof, glucosamine or a derivative thereof, L-glycine or a derivative thereof, vitamin B₁₂ or a derivative thereof, and a pharmaceutically acceptable carrier, diluent or excipient.

Suitably, according to the aforementioned aspect, the pharmaceutical composition includes methylsulfonylmethane, glucosamine, L-glycine, and vitamin B₁₂.

In a fifth aspect, the invention provides a pharmaceutical composition for improving cognitive performance, including methylsulfonylmethane or a derivative thereof, glucosamine or a derivative thereof, L-glycine or a derivative thereof, vitamin B₁₂ or a derivative thereof, and a pharmaceutically acceptable carrier, diluent or excipient.

Suitably, according to the aforementioned aspect, the pharmaceutical composition includes methylsulfonylmethane, glucosamine, L-glycine, and vitamin B₁₂.

### BRIEF DESCRIPTION OF THE FIGURES

Figure 1. Mean BDI score for study participants at day one, day fourteen and day twenty-eight of a trial of GMGB1.
Figure 2. Percentage of participants at day one in each of the four levels of depression in the BDI test.
Figure 3. Percentage of participants at day fourteen in each of the four levels of depression in the BDI test.
Figure 4. Percentage of participants at day twenty-eight in each of the four levels of depression in the BDI test.
Figure 5. Percentage of study participants having reported the listed effect.

### DETAILED DESCRIPTION OF THE INVENTION

The present invention relates to non-infectious diseases resulting from the pro-inflammatory response, particularly to methods and uses of a nutritional supplement that includes as active agents a therapeutically effective amount of methylsulfonylmethane, glucosamine, L-glycine, and vitamin B₁₂ (or a derivative of any one thereof) for controlling inflammation to treat or prevent non-infectious diseases, particularly psychiatric disorders, mood disorders, anxiety disorders, attention deficit disorders, dementia, and stress.

Throughout this specification, unless the context requires otherwise, the words "comprise", "comprises" and "comprising" will be understood to imply the inclusion of a stated integer or group of integers but not the exclusion of any other integer or group of integers.

In one aspect, the invention provides a method for treating and/or preventing a non-infectious disease in a subject in need thereof, the method including the step of administering to the subject a therapeutically effective amount of a pharmaceutical composition including (a) methylsulfonylmethane or a derivative thereof, (b) glucosamine or a derivative thereof, (c) L-glycine or a derivative thereof, and (d) vitamin B₁₂ or a derivative thereof.

A variety of non-infectious diseases and conditions, including, for example, psychiatric disorders (*e.g.,* bipolar depression, schizophrenia and suicidal ideation); mood disorders (*e.g.,* depression); anxiety disorders; attention deficit disorders; dementia (*e.g.,* Alzheimer's disease); addictive behaviour and associated withdrawal problems; stress; anxiety; cerebral palsy; Bell's palsy; premature ageing; rheumatoid arthritis; heart disease; Parkinson's disease; type 1 and type 2 diabetes; demyelinating diseases; fibromyalgia; inflammatory bowel disease (*e.g.,* Crohn's disease); asthma; allergic rhinitis; deep vein thrombosis; and platelet aggregation, have pro-inflammatory cytokines as a primary causal agent in their presentation. Non-infectious diseases can be controlled by anti pro-inflammatory cytokine treatment. GMGB1 has an anti-inflammatory effect primarily on the cytokines TNF, IL-1 and IL-6, making it useful in the treatment of non-infectious diseases as described herein.

As used herein, "treating" (or "treat" or "treatment") refers to a therapeutic intervention that ameliorates a sign or symptom of an undesired physical or mental/emotional state, or a pathological condition, after it has begun to develop. This term includes active treatment, that is, treatment directed specifically toward improvement of a subject's physical or mental/emotional state, or pathological condition, and also includes causal treatment, that is, treatment directed toward removal of a subject's undesired physical or mental/emotional state, or pathological condition. In addition, this term includes palliative treatment, that is, treatment designed for the relief of symptoms rather than the curing of a subject's undesired physical or mental/emotional state, or pathological condition, preventive treatment, that is, treatment directed to prevention of a subject's undesired physical or mental/emotional state, or pathological condition, and supportive treatment, that is, treatment employed to supplement another specific therapy directed toward the improvement of a subject's physical or mental/emotional state, or pathological condition.

The term "ameliorating," with reference to an undesired physical or mental/emotional state, or a pathological condition, refers to any observable beneficial effect of the treatment. The beneficial effect can be evidenced, for example, by a delayed onset of symptoms of the undesired physical or mental/emotional state, or pathological condition, in a susceptible subject, a reduction in severity of some or all symptoms of the undesired physical or mental/emotional state, or pathological condition, an improvement in the overall health or well-being of the subject, or by other parameters well known in the art that are specific to the particular state or condition. It is to be understood that such treating need not be absolute to be beneficial to a subject.

As used herein, "preventing" (or "prevent" or "prevention") refers to a course of action (such as administering a therapeutically effective amount of methylsulfonylmethane, glucosamine, L-glycine, and vitamin B₁₂) initiated prior to the onset of a symptom, aspect, or characteristic of an undesired physical or mental/emotional state, or pathological condition, so as to prevent or reduce the symptom, aspect, or characteristic. It is to be understood that such preventing need not be absolute to be beneficial to a subject. A "prophylactic" treatment is a treatment administered to a subject who does not exhibit signs of an undesired physical or mental/emotional state, or a pathological condition, or exhibits only early signs, for the purpose of decreasing the risk of developing the undesired physical or mental/emotional state, or pathological condition.

In one embodiment, the invention provides a method for treating and/or preventing a mood disorder (*e.g.,* depression), an anxiety disorder (*e.g.,* a panic disorder, an obsessive-compulsive disorder, a post-traumatic stress disorder, a social phobia, specific phobias, and generalized anxiety disorder), an attention deficit disorder (*e.g.,* attention deficit hyperactivity disorder and autism), dementia (*e.g.,* Alzheimer's), or stress in a subject in need thereof, the method including the step of administering to the subject a therapeutically effective amount of a pharmaceutical composition including (a) methylsulfonylmethane or a derivative thereof, (b) glucosamine or a derivative thereof, (c) L-glycine or a derivative thereof, and (d) vitamin B₁₂ or a derivative thereof.

Mood disorders, such as depression, may be diagnosed when a subject exhibits certain symptoms of such disorders. Symptoms of a mood disorder may include: apathy, persistent feelings of sadness, feeling hopeless or helpless, having low self-esteem, feeling inadequate, excessive guilt, feelings of wanting to die, loss of interest in usual activities or activities once enjoyed, difficulty with relationships, sleep disturbances, changes in appetite or weight, decreased energy, difficulty concentrating, a decrease in the ability to make decisions, suicidal thoughts or attempts, frequent physical complaints (*e.g.,* headache, stomach ache and fatigue), running away or threats of running away from home, hypersensitivity to failure or rejection, irritability, hostility, and aggression.

Accordingly, treatment of a mood disorder according to the present invention may include eliminating or reducing the frequency or severity of any of the noted symptoms or other symptoms relied upon by a subject or a medical professional in making a diagnosis of a mood disorder. Thus, in some embodiments, the present invention includes treating a subject exhibiting at least one symptom of a mood disorder by administering to the subject methylsulfonylmethane, glucosamine, L-glycine, and vitamin B₁₂ (or a derivative of any one thereof) alone, or in combination with one or more additional active agents as described herein, in an amount effective to eliminate or reduce the frequency or severity of the symptom. In particular embodiments, the mood disorder is depression. In other embodiments, the invention may specifically be described as eliminating or reducing the frequency or severity of any one of the specific symptoms of a mood disorder as provided above.

In a specific embodiment, the method includes eliminating or reducing the frequency or severity of a specific symptom of depression. In such embodiments, the method can include administering to a subject methylsulfonylmethane, glucosamine, L-glycine, and vitamin B₁₂ (or a derivative of any one thereof) alone, or in combination with one or more additional active agents as described herein. Effectiveness of the method may be established through analysis of a treated subject, through self-reporting of the treated subject or through a diagnosis of effective treatment provided by a medical professional after evaluating the treated subject.

In other embodiments, the invention provides a method for the treatment of anxiety disorders. Specifically, the invention provides a method for the treatment of any condition classified as an anxiety disorder (including, for example, panic disorder, obsessive-compulsive disorder, post-traumatic stress disorder, social phobia, specific phobias, and generalized anxiety disorder). The methods of the invention generally comprise administering methylsulfonylmethane, glucosamine, L-glycine, and vitamin B₁₂ (or a derivative of any one thereof) alone, or in combination with one or more additional active agents as described herein, to a subject suffering from a condition of an anxiety disorder. Effectiveness of the method may be established through analysis of a treated subject, through self-reporting of the treated subject or through a diagnosis of effective treatment provided by a medical professional after evaluating the treated subject.

In further embodiments, the invention provides a method for the treatment of an attention deficit disorder, such as, for example, attention deficit hyperactivity disorder (ADHD) or autism. As with mood disorders, ADHD and autism may be diagnosed when a subject exhibits certain symptoms of the disorder, as recognised by the subject and/or a medical professional. Thus, treatment of ADHD and autism according to the present invention may include eliminating or reducing the frequency or severity of any of the noted symptoms or other symptoms relied upon by a subject or a medical professional in making a diagnosis of ADHD or autism.

Accordingly, in some embodiments, the present invention includes treating a subject exhibiting at least one symptom of an attention deficit disorder by administering to the subject methylsulfonylmethane, glucosamine, L-glycine, and vitamin B₁₂ (or a derivative of any one thereof) alone, or in combination with one or more additional active agents as described herein, in an amount effective to eliminate or reduce the frequency or severity of the symptom. In other embodiments, the invention may specifically be described as eliminating or reducing the frequency or severity of any one of the specific symptoms of ADHD or autism. In such embodiments, the method can include administering methylsulfonylmethane, glucosamine, L-glycine, and vitamin B₁₂ (or a derivative of any one thereof) alone, or in combination with one or more additional active agents as described herein. Effectiveness of the method may be established through analysis of a treated subject, through self-reporting of the treated subject or through a diagnosis of effective treatment provided by a medical professional after evaluating the treated subject.

In still further embodiments, the invention provides a method for the treatment of dementia. Dementia is a neurodegenerative disorder generally characterised as the loss of a subject's learning and cognitive abilities, and is usually accompanied by behavioural, psychological and motor symptoms. A critical element of dementia is the deficiency in short- and long-term memory, associated with difficulties in abstract thought, faulty judgment, personality change, and other impairments of higher cortical function. These impairments are generally so severe that the subject cannot maintain normal social activities or relationships. Typically, the loss of cognitive skills and memory in dementia is slow, with mental deterioration taking place over years. Dementia is most common among the elderly, and is becoming more widespread as the populations of developing countries age.

Many different dementias have been enumerated, including, for example, cortical dementia, fronto-temporal dementia, Alzheimer's dementia, lewy body dementia, progressive dementia, vascular dementia, multi-infarct dementia, drug- or alcohol-related dementia, and Parkinson's-related dementia. Dementia may also result from head injury, cardiac arrest, radiation therapy related to cancer treatment, Acquired Immunodeficiency Syndrome (AIDS), Pick's disease, and Creutzfeldt-Jakob disease, including its variant form. Dementia is usually diagnosed according to its etiology, the two most common etiologies being Alzheimer's dementia and vascular dementia (*e.g.,* following a stroke). Dementia is usually progressive and irreversible unless the etiology itself is treatable. Many subjects have more than one type of dementia. A diagnosis of dementia generally involves ruling out major depressive disorders or delirium.

In most dementias, subjects often exhibit primary cognitive symptoms as well as secondary behavioural symptoms. Cognitive symptoms may include things such as loss of memory, loss of orientation perception, loss of language abilities, and impaired judgement. Secondary or behavioural symptoms may include such things as personality and behavioural changes where the subject is aggressive or verbally agitated. Dementia is often treated with anti-psychotics, benzodiazepines, beta-blockers, selective serotonin reuptake inhibitors, anti-depressants, anti-convulsives, and dietary supplements.

Accordingly, treatment of dementia according to the present invention may include eliminating or reducing the frequency or severity of any of the noted symptoms or other symptoms relied upon by a subject or a medical professional in making a diagnosis of dementia. Thus, in some embodiments, the present invention includes treating a subject exhibiting at least one symptom of dementia by administering to the subject methylsulfonylmethane, glucosamine, L-glycine, and vitamin B₁₂ (or a derivative of any one thereof) alone, or in combination with one or more additional active agents as described herein, in an amount effective to eliminate or reduce the frequency or severity of the symptom. In other embodiments, the invention may specifically be described as eliminating or reducing the frequency or severity of any one of the specific symptoms of dementia as provided above.

In other embodiments, the invention provides a method for the treatment of stress. Specifically, the invention provides a method for the treatment of mental states associated with feelings of being overloaded and unable to cope. The methods of the invention generally comprise administering methylsulfonylmethane, glucosamine, L-glycine, and vitamin B₁₂ (or a derivative of any one thereof) alone, or in combination with one or more additional active agents as described herein, to a subject suffering from stress. Effectiveness of the method may be established through analysis of a treated subject, through self-reporting of the treated subject or through a diagnosis of effective treatment provided by a medical professional after evaluating the treated subject.

In another aspect, the invention provides a method for improving cognitive performance in a subject, the method including the step of administering to the subject a therapeutically effective amount of a pharmaceutical composition including (a) methylsulfonylmethane or a derivative thereof, (b) glucosamine or a derivative thereof, (c) L-glycine or a derivative thereof, and (d) vitamin B₁₂ or a derivative thereof.

As used herein, "improving cognitive performance" includes increasing and/or enhancing a quality or condition associated with one or more cognitive abilities or skills, for example, problem solving, memory, orientation perception, language, and judgement.

The term "subject" includes both human and veterinary subjects. For example, administration to a subject can include administration to a human subject or a veterinary subject. Preferably, the subject is a human.

By "administration" is intended the introduction of a composition (*e.g.,* a pharmaceutical composition) into a subject by a chosen route.

The term "therapeutically effective amount" describes a quantity of a specified agent sufficient to achieve a desired effect in a subject being treated with that agent. For example, this may be the amount of a pharmaceutical composition comprising methylsulfonylmethane, glucosamine, L-glycine, and vitamin B₁₂ (or a derivative of any one thereof) necessary to treat or prevent an undesired physical or mental/emotional state, or a pathological condition, or for improving cognitive performance. In some embodiments, a "therapeutically effective amount" is an amount sufficient to reduce or eliminate a symptom of a non-infectious disease resulting from the pro-inflammatory response, and/or an amount sufficient to achieve a desired biological effect.

Ideally, a therapeutically effective amount of an agent is an amount sufficient to effect the desired result without causing a substantial cytotoxic effect in the subject. The effective amount of an agent useful for treating or preventing an undesired physical or mental/emotional state, or a pathological condition, will be dependent on the subject being treated, the type and severity of the state or condition, and the manner of administration of the therapeutic composition.

Toxicity and therapeutic efficacy of a treatment, such as methylsulfonylmethane, glucosamine, L-glycine, and vitamin B₁₂, can be determined by standard pharmaceutical procedures in cell cultures or experimental animals, for example, by determining the LD₅₀ (the dose lethal to 50% of the population) and/or the ED₅₀ (the dose therapeutically effective in 50% of the population). The dose ratio between toxic and therapeutic effects is the therapeutic index and it may be expressed, for example, as the ratio LD₅₀/ED₅₀. A combination of methylsulfonylmethane, glucosamine, L-glycine, and vitamin B₁₂ (or a derivative of any one thereof) that exhibit large therapeutic indices are useful.

A therapeutically effective amount of a pharmaceutical composition comprising methylsulfonylmethane, glucosamine, L-glycine, and vitamin B₁₂ (or a derivative of any one thereof) may be administered in a single dose, or in several doses, for example daily, during a course of treatment. However, the frequency of administration is dependent on the preparation applied, the subject being treated, the severity and type of undesired physical or mental/emotional state, or pathological condition, and the manner of administration of the therapy or composition.

The term "methylsulfonylmethane" refers to an organosulfur compound with the formula (CH₃)₂SO₂. It is also known as dimethyl sulfone, DMSO₂, methyl sulfone, methylsulfonylmethane, and sulfonylbismethane.

The term "glucosamine" describes a natural compound that is found in healthy cartilage. Glucosamine sulfate is a normal constituent of glycoaminoglycans in cartilage matrix and synovial fluid.

The term "L-glycine" refers to is the smallest of the 20 amino acids commonly found in proteins. It is also known as glycine, aminoethanoic acid and aminoacetic acid.

The term "vitamin B₁₂" describes cobalamin or cyanocobalamin, a member of the vitamin B complex that contains cobalt.

Biologically active variants of the various compounds disclosed herein as active agents are particularly also encompassed by the invention. Such variants should retain the general biological activity of the original compounds; however, the presence of additional activities would not necessarily limit the use thereof in the present invention. Such activity may be evaluated using standard testing methods and bioassays recognizable by the skilled artisan in the field as generally being useful for identifying such activity.

By "derivative" of methylsulfonylmethane, glucosamine, L-glycine, or vitamin B₁₂ is meant a molecule that differs in chemical structure from its parent compound, for example a homolog (differing by an increment in the chemical structure, such as a difference in the length of an alkyl chain), a molecular fragment, a structure that differs by one or more functional groups, or a change in ionization. Structural analogs are often found using quantitative structure activity relationships (QSAR), with techniques such as those disclosed in Remington: The Science and Practice of Pharmacy, 19th Edition, Chapter 28, 1995. A derivative may also be termed an "analogue".

For example, analogues of glucosamine encompassed by the present invention include, without limitation, glucosamine sulphate, N-acetyl glucosamine and quatemized amino glucosamine.

The forms of vitamin B₁₂ may be varied as known to one of ordinary skill in the art. For example, cobalamin may be present, at least partially, as methyl cobalamin or hydroxycobalamin.

Furthermore, one or more of the various compounds disclosed herein as active agents may also be present in a chelated form. In one embodiment, amino acid chelates are used. The chelated forms facilitate a more effective absorption and increased biological activities as well as increased shelf life.

The compounds described herein as active agents can also be in the form of an ester, amide, salt, solvate, prodrug, or metabolite provided they maintain pharmacological activity according to the present invention. Esters, amides, salts, solvates, prodrugs, and other derivatives of the compounds of the present invention may be prepared according to methods generally known in the art, such as, for example, those methods described by J. March, Advanced Organic Chemistry: Reactions, Mechanisms and Structure, 4th Ed. (New York: Wiley-Interscience, 1992), which is incorporated herein by reference.

Examples of pharmaceutically acceptable salts of the compounds useful according to the invention include acid addition salts. Salts of non-pharmaceutically acceptable acids, however, may be useful, for example, in the preparation and purification of the compounds. Suitable acid addition salts according to the present invention include organic and inorganic acids. Preferred salts include those formed from hydrochloric, hydrobromic, sulfuric, phosphoric, citric, tartaric, lactic, pyruvic, acetic, succinic, fumaric, maleic, oxaloacetic, methanesulfonic, ethanesulfonic, p-toluenesulfonic, benzesulfonic, and isethionic acids. Other useful acid addition salts include propionic acid, glycolic acid, oxalic acid, malic acid, malonic acid, benzoic acid, cinnamic acid, mandelic acid, salicylic acid, and the like. Particular example of pharmaceutically acceptable salts include, but are not limited to, sulfates, pyrosulfates, bisulfates, sulfites, bisulfites, phosphates, monohydrogenphosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxyenzoates, phthalates, sulfonates, xylenesulfonates, phenylacetates, phenylpropionates, phenylbutyrates, citrates, lactates, γ-hydroxybutyrates, glycolates, tartrates, methanesulfonates, propanesulfonates, naphthalene-1-sulfonates, naphthalene-2-sulfonates, and mandelates.

An acid addition salt may be reconverted to the free base by treatment with a suitable base. Preparation of basic salts of acid moieties which may be present on a compound useful according to the present invention may be prepared in a similar manner using a pharmaceutically acceptable base, such as sodium hydroxide, potassium hydroxide, ammonium hydroxide, calcium hydroxide, triethylamine, or the like.

Esters of the active agent compounds according to the present invention may be prepared through functionalization of hydroxyl and/or carboxyl groups that may be present within the molecular structure of the compound. Amides and prodrugs may also be prepared using techniques known to those skilled in the art. For example, amides may be prepared from esters, using suitable amine reactants, or they may be prepared from anhydride or an acid chloride by reaction with ammonia or a lower alkyl amine. Moreover, esters and amides of compounds of the invention can be made by reaction with a carbonylating agent (*e.g.,* ethyl formate, acetic anhydride, methoxyacetyl chloride, benzoyl chloride, methyl isocyanate, ethyl chloroformate, methanesulfonyl chloride) and a suitable base (*e.g.,* 4-dimethylaminopyridine, pyridine, triethylamine, potassium carbonate) in a suitable organic solvent (*e.g.,* tetrahydrofuran, acetone, methanol, pyridine, N,N-dimethylformamide) at a temperature of 0 °C to 60 °C. Prodrugs are typically prepared by covalent attachment of a moiety, which results in a compound that is therapeutically inactive until modified by an individual's metabolic system. Examples of pharmaceutically acceptable solvates include, but are not limited to, compounds according to the invention in combination with water, isopropanol, ethanol, methanol, DMSO, ethyl acetate, acetic acid, or ethanolamine.

In the case of solid compositions, it is understood that the compounds used in the methods of the invention may exist in different forms. For example, the compounds may exist in stable and metastable crystalline forms and isotropic and amorphous forms, all of which are intended to be within the scope of the present invention.

If a compound useful as an active agent according to the invention is a base, the desired salt may be prepared by any suitable method known to the art, including treatment of the free base with an inorganic acid, such as hydrochloric acid, hydrobromic acid, sulfuric acid, nitric acid, phosphoric acid and the like, or with an organic acid, such as acetic acid, maleic acid, succinic acid, mandelic acid, fumaric acid, malonic acid, pyruvic acid, oxalic acid, glycolic acid, salicylic acid, pyranosidyl acids such as glucuronic acid and galacturonic acid, alpha-hydroxy acids such as citric acid and tartaric acid, amino acids such as aspartic acid and glutamic acid, aromatic acids such as benzoic acid and cinnamic acid, sulfonic acids such a p-toluenesulfonic acid or ethanesulfonic acid, or the like.

If a compound described herein as an active agent is an acid, the desired salt may be prepared by any suitable method known to the art, including treatment of the free acid with an inorganic or organic base, such as an amine (primary, secondary or tertiary), an alkali metal or alkaline earth metal hydroxide or the like. Illustrative examples of suitable salts include organic salts derived from amino acids such as glycine and arginine, ammonia, primary, secondary and tertiary amines, and cyclic amines such as piperidine, morpholine and piperazine, and inorganic salts derived from sodium, calcium, potassium, magnesium, manganese, iron, copper, zinc, aluminium and lithium.

The present invention further includes prodrugs and active metabolites of the active agent compounds described herein. Any of the compounds described herein can be administered as a prodrug to increase the activity, bioavailability, or stability of the compound or to otherwise alter the properties of the compound. Typical examples of prodrugs include compounds that have biologically labile protecting groups on a functional moiety of the active compound. Prodrugs include compounds that can be oxidized, reduced, aminated, deaminated, hydroxylated, dehydroxylated, hydrolyzed, dehydrolyzed, alkylated, dealkylated, acylated, deacylated, phosphorylated, and/or dephosphorylated to produce the active compound. In preferred embodiments, the compounds of this invention possess anti-proliferative activity against abnormally proliferating cells, or are metabolized to a compound that exhibits such activity.

A number of prodrug ligands are known. In general, alkylation, acylation, or other lipophilic modification of one or more heteroatoms of the compound, such as a free amine or carboxylic acid residue, reduces polarity and allows passage into cells. Examples of substituent groups that can replace one or more hydrogen atoms on the free amine and/or carboxylic acid moiety include, but are not limited to, the following: aryl; steroids; carbohydrates (including sugars); 1,2-diacylglycerol; alcohols; acyl (including lower acyl); alkyl (including lower alkyl); sulfonate ester (including alkyl or arylalkyl sulfonyl, such as methanesulfonyl and benzyl, wherein the phenyl group is optionally substituted with one or more substituents as provided in the definition of an aryl given herein); optionally substituted arylsulfonyl; lipids (including phospholipids); phosphotidylcholine; phosphocholine; amino acid residues or derivatives; amino acid acyl residues or derivatives; peptides; cholesterols; or other pharmaceutically acceptable leaving groups which, when administered in vivo, provide the free amine and/or carboxylic acid moiety. Any of these can be used in combination with the disclosed active agents to achieve a desired effect.

Various combinations of one or more additional active agents as known by one of skill in the art for the treatment and/or prevention of non-infectious diseases and conditions may be administered to a subject in need thereof in addition to a therapeutically effective amount of methylsulfonylmethane, glucosamine, L-glycine, and vitamin B₁₂ (or a derivative of any one thereof). That is, one or more additional active agents traditionally used for the treatment and/or prevention of psychiatric disorders, mood disorders, anxiety disorders, attention deficit disorders, dementia, addictive behaviour and associated withdrawal problems, stress, anxiety, cerebral palsy, Bell's palsy, premature ageing, rheumatoid arthritis, heart disease, Parkinson's disease, type 1 and type 2 diabetes, demyelinating diseases, fibromyalgia, inflammatory bowel disease, asthma, allergic rhinitis, deep vein thrombosis, and platelet aggregation may be administered to a subject in addition to a therapeutically effective amount of methylsulfonylmethane, glucosamine, L-glycine, and vitamin B₁₂ (or a derivative of any one thereof).

For example, a norepinephrine reuptake inhibitor, a selective serotonin reuptake inhibitor, a tricyclic antidepressant, and/or a monoamine oxidase inhibitor can be administered with methylsulfonylmethane, glucosamine, L-glycine, and vitamin B₁₂ (or a derivative of any one thereof) in certain embodiments for treating and/or preventing a mood disorder, an anxiety disorder, an attention deficit disorder, dementia, or stress, or for improving cognitive performance.

In certain embodiments, the one or more additional active agents provide a conserving effect on the methylsulfonylmethane, glucosamine, L-glycine, and vitamin B₁₂. In further embodiments, the methylsulfonylmethane, glucosamine, L-glycine, and vitamin B₁₂ provide a conserving effect on the one or more additional active agents. In still further embodiments, the one or more additional active agents provide a complimentary effect to the action of the methylsulfonylmethane, glucosamine, L-glycine, and vitamin B₁₂, preferably eliminating or reducing the frequency or severity of one or more symptoms associated with a non-infectious disease.

By "reducing", as in reducing the frequency or severity of one or more of the symptoms (including specific symptoms) associated with a non-infectious disease, is meant a lessening or shortening of a symptom, aspect, or characteristic associated with a non-infectious disease, or of the length of time a subject experiences a symptom, aspect, or characteristic associated with a non-infectious disease. It is to be understood that such reducing need not be absolute to be beneficial to a subject.

Norepinephrine reuptake inhibitors are also known or noradrenalin reuptake inhibitors, and generally function to elevate the level of norepinephrine in the central nervous system by inhibiting reuptake of norepinephrine from the synaptic cleft into the presynaptic neuronal terminal. Norepinephrine is a catecholamine and phenylethylamine that functions as a neurotransmitter and is known to affect many conditions. The term "norepinephrine reuptake inhibitor" includes any compound typically recognised as inhibiting the reuptake of norepinephrine in the central nervous system. Non-limiting examples of norepinephrine reuptake inhibitors useful according to the invention include atomoxetine (STRATTERA®), reboxetine (EDRONAX®, VESTRA® or NOREBOX®), viloxazine (EMOVIT®, VIVALAN®, VIVARINT®, or VIVILAN®), maprotiline (DEPRILEPT®, LUDIOMIL® or PSYMION®), bupropion (WELLBUTRIN® or ZYBAN®), and radafaxine.

Non-limiting examples of specific selective serotonin reuptake inhibitors useful according to the invention include fluoxetine (PROZAC®), paroxetine (PAXIL®), citalopram (CELEXA®), escitalopram (LEXAPRO®), fluvoxamine (LUVOX®), and sertraline (ZOLOFT®).

Tricyclic antidepressants are a class of antidepressant compounds that can be described as including any compound exhibiting antidepressant activity and having a chemical formula including a fused three ring structure. Exemplary tricyclic antidepressants for use according to the present invention include, but are not limited to, amitriptyline (ELAVIL®), amoxapine, butriptyline, clomipramine (ANAFRANIL®), desipramine (NORPRAMIN®), dibenzepin, dosulepin, doxepin (SINEQUAN®), imipramine (TOFRANIL®), lofepramine, nortriptyline (PAMELOR® or AVENTYL®), protriptyline (VIVACTYL®), and trimipramine (SURMONTIL®).

Monoamine oxidase inhibitors include a class of compounds understood to act by inhibiting the activity of monoamine oxidase, an enzyme generally found in the brain and liver, which functions to break down monoamine compounds, typically through deamination.

There are two isoforms of monoamine oxidase inhibiting compounds, MAO-A and MAO-B. The MAO-A isoform preferentially deaminates monoamines typically occurring as neurotransmitters (*e.g.,* serotonin, melatonin, epinephrine, norepinephrine, and dopamine). Thus, monoamine oxidase inhibitors have been historically prescribed as antidepressants and for treatment of other social disorders, such as agoraphobia and social anxiety. The MAO-B isoform preferentially deaminates phenylethylamine and trace amines. Dopamine is equally deaminated by both isoforms. Monoamine oxidase inhibitors may by reversible or non-reversible and may be selective for a specific isoform. For example, the monoamine oxidase inhibitor moclobemide (also known as Manerix or Aurorix) is known to be approximately three times more selective for MAO-A than MAO-B.

Any compound generally recognized as being a monoamine oxidase inhibitor may be useful according to the present invention. Non-limiting examples of monoamine oxidase inhibitors useful in combination with methylsulfonylmethane, glucosamine, L-glycine, and vitamin B₁₂ (or a derivative of any one thereof) for preparing compositions according to the invention include the following: isocarboxazid (MARPLAN®), moclobemide (Aurorix, Manerix or Moclodura), phenelzine (NARDIL®), tranylcypromine (PARNATE®), selegiline (ELDEPRYL®, EMSAM® or 1-deprenyl), lazabemide, nialamide, iproniazid (marsilid, iprozid, ipronid, rivivol, or propilniazida), iproclozide, toloxatone, harmala, brofaromine (Consonar), benmoxin (Neuralex), and certain tryptamines, such as 5-MeO-DMT (5-Methoxy-N,N-dimethyltryptamine) or 5-MeO-AMT (5-methoxy-α-methyltryptamine).

In some embodiments, the combination of methylsulfonylmethane, glucosamine, L-glycine, and vitamin B₁₂ (or a derivative of any one thereof) and one or more additional active agents produces a synergistic effect in the treatment and/or prevention of a non-infectious disease. Accordingly, the present invention also includes a method of enhancing the therapeutic effectiveness of an active agent in treating any condition for which such agents are used.

In one embodiment, the methylsulfonylmethane, glucosamine, L-glycine, and vitamin B₁₂ (or a derivative of any one thereof) are administered prior to the administration of the one or more additional active agents. In another embodiment, the methylsulfonylmethane, glucosamine, L-glycine, and vitamin B₁₂ are administered after the administration of the one or more additional active agents. In still another embodiment, the methylsulfonylmethane, glucosamine, L-glycine, and vitamin B₁₂ are administered simultaneously with the administration of the one or more additional active agents. In yet another embodiment, administration of the methylsulfonylmethane, glucosamine, L-glycine, and vitamin B₁₂ and the administration of the one or more additional active agents (either sequentially or concurrently) results in an improvement in an undesired physical or mental/emotional state, or a pathological condition, that is greater than such an improvement from administration of either the methylsulfonylmethane, glucosamine, L-glycine, and vitamin B₁₂ or one or more additional active agents in the absence of the other.

The active agents of the present disclosure can be administered by any conventional method available for use in conjunction with pharmaceutical compositions. The pharmaceutical compositions of the present disclosure may be administered alone or may be administered with additional active agents if desired.

The pharmaceutical compositions described can be used in the form of a medicinal preparation, for example, in aerosol, solid, semi-solid, or liquid form which contains the methylsulfonylmethane, glucosamine, L-glycine, and vitamin B₁₂ (or a derivative of any one thereof) disclosed as active ingredients. In addition, the compositions may be used in an admixture with an appropriate pharmaceutically acceptable carrier. Such pharmaceutically acceptable carriers include, but are not limited to, organic or inorganic carriers, excipients or diluents suitable for pharmaceutical applications. The active ingredients may be compounded, for example, with the usual non-toxic pharmaceutically acceptable carriers, excipients or diluents for tablets, pellets, capsules, inhalants, suppositories, solutions, emulsions, suspensions, aerosols, and any other form suitable for use.

Pharmaceutically acceptable carriers for use in pharmaceutical compositions are well known in the art, and are described, for example, in Remington: The Science and Practice of Pharmacy Pharmaceutical Sciences, Lippincott Williams and Wilkins (A. R. Gennaro editor, 20th edition). Such materials are nontoxic to the recipients at the dosages and concentrations employed and include, but are not limited to, water, talc, gum acacia, gelatin, magnesium trisilicate, keratin, colloidal silica, urea, buffers such as phosphate, citrate, acetate, and other organic acid salts, antioxidants such as ascorbic acid, peptides, low molecular weight (less than about ten residues) peptides such as, but not limited to, polyarginine, proteins, such as, but not limited to, serum albumin, gelatin, or immunoglobulins, hydrophilic polymers such as, but not limited to, polyvinylpyrrolidinone, amino acids such as, but not limited to, glycine, glutamic acid, aspartic acid, or arginine, monosaccharides, disaccharides, and other carbohydrates including cellulose or its derivatives, lactose, mannitol, glucose, mannose, dextrins, potato or corn starch or starch paste, chelating agents such as, but not limited to, EDTA, sugar alcohols such as mannitol or sorbitol, counterions such as, but not limited to, sodium, and nonionic surfactants such as, but not limited to, the Tweens, Pluronics or polyethyleneglycol. In addition, the compositions may comprise auxiliary agents, such as, but not limited to, taste-enhancing agents, stabilizing agents, thickening agents, colouring agents and the like.

The pharmaceutical compositions may be prepared for storage or administration by mixing the active ingredients, each having a desired degree of purity, with physiologically acceptable carriers, excipients, stabilizers, auxiliary agents, and the like, as is known in the art. Such compositions may be provided in sustained release or timed release formulations.

The pharmaceutical compositions containing the active ingredients may be administered orally in solid dosage forms, such as capsules, tablets, and powders, or in liquid dosage forms, such as elixirs, syrups and suspensions. Furthermore the compositions containing the active ingredients may be administered parenterally, in sterile liquid dosage forms, by transmucosal delivery via solid, liquid or aerosol forms or transdermally via a patch mechanism or ointment. Various types of transmucosal administration include respiratory tract mucosal administration, nasal mucosal administration, oral transmucosal (such as sublingual and buccal) administration, and rectal transmucosal administration.

For preparing solid compositions such as, but not limited to, tablets or capsules, the methylsulfonylmethane, glucosamine, L-glycine, and vitamin B₁₂ (or a derivative of any one thereof) described may be mixed with appropriate pharmaceutically acceptable carriers, such as conventional tableting ingredients (e.g., lactose, sucrose, mannitol, corn starch, potato starch, alginic acid, microcrystalline cellulose, acacia, gelatin, gums, colloidal silicon dioxide, croscarmellose sodium, talc, sorbitol, stearic acid magnesium stearate, calcium stearate, zinc stearate, stearic acid, and dicalcium phosphate), other excipients, colorants, diluents, buffering agents, disintegrating agents, moistening agents, preservatives, flavouring agents, and pharmacologically compatible carriers, as well as diluents (*e.g.,* water, saline or buffering solutions) to form a substantially homogenous composition. The substantially homogenous composition means the components are dispersed evenly throughout the composition so that the composition may be readily subdivided into equally effective unit dosage forms such as tablets, pills and capsules.

The solid compositions described may be coated or otherwise compounded to provide a dosage form affording the advantage of prolonged action. For example, tablets or pills can comprise an inner dosage and an outer dosage component, the latter being in the form of an envelope over the former. The two components can be separated by an enteric layer which serves to resist disintegration in the stomach and permits the inner component to pass intact through the stomach or to be delayed in release. A variety of materials can be used for such enteric layers or coatings such materials including a number of polymeric acids and mixtures of polymeric acids with such materials as shellac, cetyl alcohol and cellulose acetate.

The active ingredients may also be formulated in rectal compositions such as suppositories or retention enemas, for example, containing conventional suppository bases such as cocoa butter or other glycerides. The solid compositions may also comprise a capsule, such as hard- or soft-shelled gelatin type containing, for example, surfactants, lubricants, and inert fillers, such as lactose, sucrose, calcium phosphate, and corn starch.

For intranasal administration, intrapulmonary administration or administration by other modes of inhalation, the pharmaceutical compositions may be delivered in the form of a solution or suspension from a pump spray container or as an aerosol spray presentation from a pressurized container or nebulizer, with the use of a suitable propellant (*e.g.,* dichlorodifluoromethane, trichlorofluoromethane, dichlorotetrafluoroethane, nitrogen, propane, carbon dioxide, or other suitable gas) or as a dry powder. In the case of an aerosol or dry powder format, the amount (dose) of the composition delivered may be determined by providing a valve to deliver a metered amount.

Liquid forms may be administered orally, parenterally or via transmucosal administration. Suitable forms for liquid administration include aqueous solutions, suitably flavoured syrups, aqueous or oil suspensions, and flavoured emulsions with edible oils such as cottonseed oil, sesame oil, coconut oil, or peanut oil, as well as elixirs and similar pharmaceutical vehicles. Suitable dispersing or suspending agents for aqueous suspensions include synthetic natural gums, such as tragacanth, acacia, alginate, dextran, sodium carboxymethyl cellulose, methylcellulose, polyvinylpyrrolidone, and gelatin. Such liquid preparations may be prepared by conventional means with pharmaceutically acceptable additives such as suspending agents (*e.g.,* sorbitol syrup, methyl cellulose or hydrogenated edible fats), emulsifying agents (*e.g.,* lecithin or acacia), non-aqueous vehicles (*e.g.,* almond oil, oily esters or ethyl alcohol), preservatives (*e.g.,* methyl or propyl p-hydroxybenzoates or sorbic acid), and artificial or natural colours and/or sweeteners.

Liquid formulations may include diluents, such as water and alcohols (*e.g.,* ethanol, benzyl alcohol, propylene glycol, glycerin, and the polyethylene alcohols), either with or without the addition of a pharmaceutically acceptable surfactant, suspending agent, or emulsifying agent.

For buccal or sublingual administration, the pharmaceutical composition may take the form of tablets or lozenges formulated in conventional manners. Lozenge forms can comprise the active ingredient in a flavour, usually sucrose and acacia or tragacanth, as well as pastilles comprising the active ingredient in an inert base, such as gelatin and glycerin, or sucrose and acadia, emulsions, and gels containing, in addition to the active ingredient, such carriers as are known in the art.

The pharmaceutical compositions may be formulated for parenteral administration. Parenteral administration includes, but is not limited to, intravenous administration, subcutaneous administration, intramuscular administration, intradermal administration, intrathecal administration, intraarticular administration, intracardiac administration, retrobulbar administration, and administration via implants, such as sustained release implants.

The pharmaceutical compositions may be presented in unit-dose or multidose sealed containers, such as ampules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets. The requirements for effective pharmaceutically acceptable carriers for injectable compositions are well known in the art.

Therapeutic treatments can include a therapeutically effective amount of methylsulfonylmethane, glucosamine, L-glycine, and vitamin B₁₂ (or a derivative of any one thereof) necessary to prevent or treat an undesired physical or mental/emotional state, or a pathological condition, or for improving cognitive performance. Ideally, a therapeutically effective amount of an agent is an amount sufficient to effect the desired result without causing a substantial cytotoxic effect in the subject. The effective amount of an agent useful for preventing or treating an undesired physical or mental/emotional state, or a pathological condition, or for improving cognitive performance, will be dependent on the subject being treated, the severity of the state or condition, and the manner of administration of the therapeutic composition. Effective amounts can be determined by standard clinical techniques.

For example, when administering a pharmaceutical composition comprising methylsulfonylmethane, glucosamine, L-glycine, and vitamin B₁₂ (or a derivative of any one thereof), the precise dose to be employed in the formulation will depend on the route of administration, and should be decided according to the judgment of the health care practitioner and each subject's circumstances. The concentration of an active ingredient (such as methylsulfonylmethane, glucosamine, L-glycine, and vitamin B₁₂) in a topical composition (such as an ointment, cream, gel, or lotion) is typically from about 0.2% to about 1% (by weight relative to the total weight of the topical composition); for example, from about 0.3% to about 0.9%, from about 0.4% to about 0.8%, and from about 0.5% to about 0.7%. Within the ranges, higher concentrations allow a suitable dosage to be achieved while applying the lotion, ointment, gel, or cream in a lesser amount or with less frequency.

In other embodiments, a dosage range for non-topical administration (such as oral administration, or intravenous or intraperitoneal injection) of a pharmaceutical composition containing methylsulfonylmethane, glucosamine, L-glycine, and vitamin B₁₂ is from about 0.1 to about 200 mg/kg body weight for each active agent in single or divided doses; for example from about 1 to about 100 mg/kg, from about 2 to about 50 mg/kg, from about 3 to about 25 mg/kg, or from about 5 to about 10 mg/kg.

Acceptable daily dosages of the active ingredients (*i.e*., methylsulfonylmethane, glucosamine, L-glycine, and vitamin B₁₂) of the pharmaceutical compositions of the present invention include between 500 and 6,000 mg of methylsulfonylmethane (*e.g.,* 800 mg, 1,000 mg, 1,500 mg, 2,000 mg, 2,500 mg, 3,000 mg, 3,500 mg, 4,000 mg, 4,500 mg, 5,000 mg, and 5,500 mg), between 500 and 6,000 mg of glucosamine (*e.g.,* 800 mg, 1,000 mg, 1,500 mg, 2,000 mg, 2,500 mg, 3,000 mg, 3,500 mg, 4,000 mg, 4,500 mg, 5,000 mg, and 5,500 mg), between 500 and 2,000 mg of L-glycine (*e.g.,* 800 mg, 1,000 mg and 1,500 mg), and between 0.02 and 2.0 mg of vitamin B₁₂ (*e.g.,* 0.02 mg, 0.04 mg, 0.06 mg, 0.08 mg, 0.2 mg, 0.4 mg, 0.6 mg, 0.8 mg,1.0 mg, 1.2 mg, and 1.5 mg).

The pharmaceutical compositions of the present disclosure can be administered at about the same dose throughout a treatment period, in an escalating dose regimen, or in a loading-dose regime (for example, in which the loading dose is about two to five times the maintenance dose). In some embodiments, the dose is varied during the course of a treatment based on the condition of the subject being treated, the severity of the migraine, the apparent response to the therapy, and/or other factors as judged by one of ordinary skill in the art. In some embodiments long-term treatment with a disclosed pharmaceutical composition is contemplated.

In yet another aspect, the invention provides use of methylsulfonylmethane or a derivative thereof, glucosamine or a derivative thereof, L-glycine or a derivative thereof, and vitamin B₁₂ or a derivative thereof in the manufacture of a medicament for treating a non-infectious disease in a subject.

In further aspects, the invention provides a pharmaceutical composition for treating or preventing a non-infectious disease, or for improving cognitive performance, including methylsulfonylmethane or a derivative thereof, glucosamine or a derivative thereof, L-glycine or a derivative thereof, vitamin B₁₂ or a derivative thereof, and a pharmaceutically acceptable carrier, diluent or excipient.

In one embodiment, the pharmaceutical composition includes between 500 and 6,000 mg of methylsulfonylmethane (*e.g.,* 800 mg, 1,000 mg, 1,500 mg, 2,000 mg, 2,500 mg, 3,000 mg, 3,500 mg, 4,000 mg, 4,500 mg, 5,000 mg, and 5,500 mg), between 500 and 6,000 mg of glucosamine (*e.g.,* 800 mg, 1,000 mg, 1,500 mg, 2,000 mg, 2,500 mg, 3,000 mg, 3,500 mg, 4,000 mg, 4,500 mg, 5,000 mg, and 5,500 mg), between 500 and 2,000 mg of L-glycine (*e.g.,* 800 mg, 1,000 mg and 1,500 mg), and between 0.02 and 2.0 mg of vitamin B₁₂ (*e.g.,* 0.02 mg, 0.04 mg, 0.06 mg, 0.08 mg, 0.2 mg, 0.4 mg, 0.6 mg, 0.8 mg,1.0 mg, 1.2 mg, and 1.5 mg).

So that the present invention may be more readily understood and put into practical effect, the skilled person is referred to the following non-limiting examples.

### EXAMPLES

### Example1: Initial study

Because of the complicated monitoring required for most of the potential non-infectious diseases that could be involved with the study, it was considered that an assessment of improvement in depression using the GMGB1 would be a simple starting point. Although the study was limited by size and the fact that only qualitative data was being collected, a good response at this stage would be encouraging. Quantitative data using the Beck Depression Index (BDI), a standard tool that assesses the degree of depression, was used retrospectively on participants.

The BDI score indicates four levels of depression: (i) a score of 0 to 9 indicates that there is minimal or no evidence of depression; (ii) a score of 10 to 16 indicates evidence of mild depression; (iii) a score of 17 to 29 indicates moderate depression; and (iv) a score of 30 to 63 indicates severe depression.

The aim of the study was to assess if psychiatric illness is caused by the presence of large amounts pro-inflammatory cytokines, mainly TNF. It would achieve this by assessing if the treatment with GMGB1 would make a positive change in the participants presentations. The study included only a few people (n = 9), but was useful in that it showed trends indicating a more detailed study would be beneficial. Participants all willingly agreed to participate in the study and gave informed consent.

All of the components in GMGB1 are of low molecular weight, so bioavailability is very good and as a consequence they can move in and out of all tissues. Half-life indicated we could deliver the treatment in a once-a-day compound which would improve compliance. The formula for the study, based on the ratio of molecular weights, was 1200mg of glucosamine analog or 400 mg of quaternized amino glucosamine, 400mg of L-glycine, 500mg of methylsulfonylmethane, and 250µg of vitamin B₁₂.

### Results

Patient 1: Initial report was that she was sad, heavy of heart and overwhelmed by everything; unable to make decisions, angry, see negative in everything, and unable to move forward in life. After roughly ten days of treatment, she was happier, more carefree, less easily upset, calmer, relaxed, and found issues not so important anymore, less worried, having the ability to move on. The results were still continuing eight weeks after commencement.

Patient 2: Was a bipolar patient on no medications for bipolar disorder, but on anti depressives. A typical problem with bipolar treatments is that they make you "flat" - feeling neither good nor bad. He commented that with this regime he was having none of the mood swings he is used to, but without the "flat" feeling; he felt normal.

Patient 3: A lady with Bell's palsy who has improved daily since she started the treatment.

Patient 4: A depressed male on medication for depression, but still depressed. Since going on the regime he has felt normal for the first time in a long time.

Patient 5: A depressed person, stressed, anxious and crying every day, and saw no future. After the treatment, now stable and has a positive view of life.

Patient 6: A very busy person who suffered from stress and was predisposed to depression. He reported that the treatment was doing good things for his emotional and mental well being, and he is less stressed and able to cope with his busy schedule.

Patient 7: A sceptical clinician started the regime, and after one week reported his mood had improved significantly.

Initial results from the preliminary study with GMGB1 on volunteer patients with major depression showed a positive outcome from the treatment. Using the Beck Depression Index as a measurement tool, all patients screened between 20 and 45 with this tool prior to beginning the treatment regime. This indicated a range of moderate to severe depression. All patients responded by reducing their score to less than 5 after two weeks of treatment with GMGB1, indicating that they were no longer depressed.

All but three of these patients had previously been on psychotropic medications and had never experienced this degree of benefit before. The most common comment was clarity of thought and improved cognitive capacity. Even those on selective serotonin reuptake inhibitor medication had significant improvement and the "muzzy" head disappeared. The other common comment was that of being stable, they did not cycle between mania and depression as before, even in the presence of triggers that would have made them feel depressed before.

An initial result from a patient who was part of the initial study was encouraging in this regard. Coincidental to the depression, the patient suffered for many years from what appeared to be a demyelinating disease characterised by hyper-reflexivity and claw toes in both feet. A treatment for claw toe associated with fibromyalgia is selective serotonin reuptake inhibitor medication. This person has been treated with a selective serotonin reuptake inhibitor for more than eight years with no resolution to the problem. Since using GMGB1 however, both feet have resolved, indicating a probable use for this compound in neuromuscular diseases and fibromyalgia in general.

A person in the study who had been on a selective serotonin reuptake inhibitor and Valium treatment for many years reduced their intake of these medications abruptly. Normally, the cessation of these medications would require a period of several months to stop because of the alarming withdrawal effects. Taking GMGB1 at the time of the cessation enabled the person to stop the medications without any adverse effects, indicating a benefit in its use in withdrawal from similar drugs or drugs of abuse.

### Example 2: Efficacy of GMGB1 as a mood stabiliser in individuals with certain depressive and anxiety disorders

As a result of encouraging results from the initial small trial of GMGB1, a larger and more rigorous study was planned. This study was run from a general practitioner's surgery and the patients were selected on an "as presenting" basis. It was initially planned that in the first protocol there would be a placebo arm. However, as the potential participants were coming to the general practitioner's surgery for treatment, it was felt that it would be unethical to not provide a potentially beneficial treatment.

All participants provided they were eligible for inclusion in the study as per the protocol, were informed about all aspects of the study and completed an informed consent form. Each participant was allocated a unique number that would be used for identifying results and maintaining confidentiality.

Thirty-nine participants were involved with the study. Of these, four had no response and two withdrew from the study.

The efficacy of the treatment was monitored primarily using the Beck Depression Index. Changes from the initial score were assessed, on day one of the study, and at day fourteen and day twenty-eight. Efficacy was assessed using a patient diary and an effects record sheet. A number of different effects were noted by the participants towards the end of the study. All effects were put together on a check sheet and participants completed this in addition to their diaries.

### Results

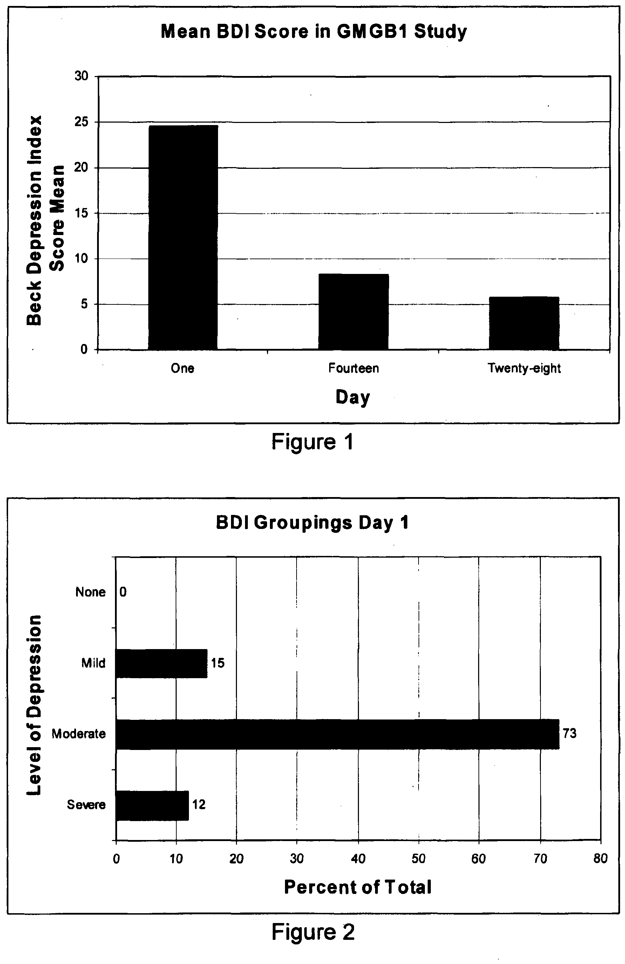

Analysis of the BDI data revealed on day one a mean of 24.6 with a standard deviation of 9.6. On day fourteen the mean was 8.3 with a standard deviation of 6.3, and on day twenty-eight the mean was 5.8 with a standard deviation of 4.4 (Figure 1). Statistical analysis of the difference between day one and day twenty-eight gave a p value of <0.001.

At the start of the study, 24% of the participants were severely depressed, 71% were moderately depressed, 5% were mildly depressed, and nil were in the category of no depression (Figure 2).

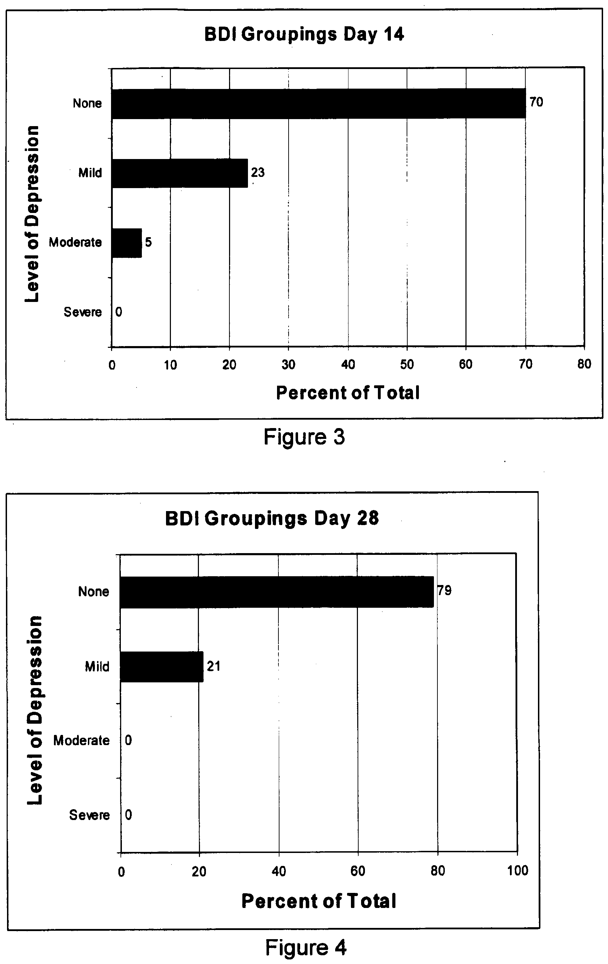

Figures 3 and 4 illustrate the changes seen at fourteen and twenty-eight days, respectively, and it can be seen that the group of participants ended up with 73% in the "none" or minimal evidence of depression, with zero severely affected by depression.

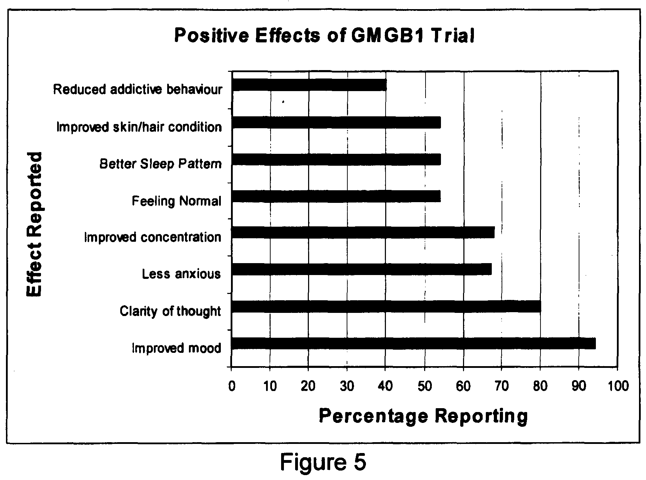

During the study, participants were asked to record any effects of the treatment (either positive or negative) that might give an indication of the effectiveness of GMGB1. There were a number of unexpected effects that indicate further areas of study (Figure 5; Table I).

The results revealed a dramatic change in the intensity of depression during the twenty-eight days of the study. The data shows that the participants involved in the study had an improvement in their BDI results of 68%. This is a significant individual improvement, as it is considered that any improvement over 50% is a significant clinical improvement.

Those effects reported by participants indicate GMGB1 is very useful in improving mood and giving participants a clear head. A role in age-related dementia is indicated. One participant (greater than 80 years old) had much improved concentration and clarity of thought. More than 60% of participants reported improved concentration, lower anxiety, improved clarity of thought, and improved mood, making this an important treatment across ages and genders.

Twelve individuals elected to continue with the trial for at least six months after the completion of the twenty-eight day study. During this period, the individuals maintained their BDI levels and reported an increased ability to cope with changes that would have put them into deep anxiety or depression in the past.

Other study participants that decided to stop the treatment after the twenty-eight day trial (for a variety of reasons) reported that the benefit they received stopped only a few days after discontinuing treatment. One participant, a lady with bipolar depression, stated that on ceasing GMGB1, within five days her depression and fibromyalgia returned. Another, who started with severe depression and recorded no depression after fourteen days, returned to having severe depression after stopping the medication for fourteen days.

A number of euthymic individuals, volunteering to be included to assess the ability to modify stressful behaviour, reported a significant and continued benefit for periods of up to twelve months whilst taking GMGB1. These benefits diminished two to three days following cessation of taking GMGB1, and individuals experienced a recurrence of their mood fluctuation, which again resolved on continuing with GMGB1.

Several effects reported were only relevant to those experiencing the problem that was alleviated in the first place. As an example, not all participants suffered from skin conditions, but all of those that did had relief from using GMGB1. Excessive alcohol use and other addictive behaviour were reported by a number of participants. As an example, one female participant, who reported having 2-3 glasses of wine every night for many years, stated that she no longer had an interest in alcohol. There would appear to be a reduction in the craving associated with these addictive behaviours. Those reporting an increased libido made notes to the fact that the increase was not just a small amount but very significant. All the effects reported have been consistent with GMGB1 having a significant effect on certain contributing factors in a variety of presentations.

### Discussion

Under the EU guidelines for the assessment of antidepressants, a new medication is considered effective and having a good efficacy if the percentage of participants with a good response is over 50%. The efficacy response is defined as a difference between the baseline and post treatment score in symptomatology, but also should be expressed as the proportion of responders. In major depression, a 50% improvement on the usual rating scales is accepted as clinically relevant response.

In this study of thirty-nine people, a clinical improvement was achieved in 89% of patients to non-responders. Although there was improvement in depression, as indicated by the improvement in BDI score, and a lowering of anxiety levels, the predominant improvement was one of mood stabilisation. Even in the euthymic individuals, the fact that they reported that they did not have outbursts of anger, or negative internalisation of emotions for the duration of the trial (greater than six months), is very significant.

GMGB1 has few side effects (weight gain was reasonably common and a few people reported headaches) and as a consequence is well tolerated. This toleration enables it to be ethically used in individuals experiencing minor mood swings associated with living in a stressful environment, and can be used for an extended period of time, even prophylactically. Although there was a significant drop in BDI score in the study seen in depressed patients, this could be primarily as a result of an improvement in their ability to cope with daily stressors. The capacity of the compound to also work effectively in reducing the impact of stressors in anxiety patients makes this medication stand out from other mood stabilisers, that are associated only with depressed patients in manic states.

Throughout this specification, the aim has been to describe the preferred embodiments of the invention without limiting the invention to any one embodiment or specific collection of features. Various changes and modifications may be made to the embodiments described and illustrated herein without departing from the broad spirit and scope of the invention.

All computer programs, algorithms, patent and scientific literature referred to in this specification is incorporated herein by reference in their entirety.

**Table I. Positive Effects of GMGB1 Trial**

| ID | % | Sex | Age | Dur | Quest | BP | Cone | Stress | Mood | Anx | Sleep | Skin | Sick | Effect |
|---|---|---|---|---|---|---|---|---|---|---|---|---|---|---|
| 1 | 31 | F | 41 | 1 | 1 | 0 | 0 | 1 | 1 | 0 | 1 | 1 | 0 | 0 |
| 2 | 60 | M | 45 | 1 | 1 | 0 | 1 | 1 | 1 | 1 | 0 | 0 | 0 | 0 |
| 3 | 94 | M | 57 | 1 | 1 | 1 | 1 | I | 1 | 1 | 1 | 0 | 1 | 0 |
| 4 | 43 | M | 60 | 0 | 0 | 0 | 0 | 0 | 0 | 0 | 0 | 0 | 0 | 0 |
| 5 | 59 | F | 50 | 0 | 0 | 0 | 0 | 0 | 0 | 0 | 0 | 0 | 0 | 0 |
| 6 | 95 | F | 51 | 1 | 1 | 0 | 1 | 1 | 1 | 1 | 0 | 0 | 0 | 0 |
| 7 | 39 | M | 81 | 0 | 0 | 0 | 0 | 0 | 0 | 0 | 0 | 0 | 0 | 0 |
| 8 | 28 | F | 89 | 0 | 0 | 0 | 0 | 0 | 0 | 0 | 0 | 0 | 0 | 0 |
| 9 | 0 | M | 57 | 0 | 0 | 0 | 0 | 0 | 0 | 0 | 0 | 0 | 0 | 0 |
| 10 | 74 | M | 28 | 0 | 1 | 1 | 1 | 0 | 1 | 0 | 0 | 0 | 0 | 0 |
| 11 | 74 | F | 27 | 0 | 1 | 0 | 1 | 1 | 1 | 1 | 1 | 0 | 0 | 0 |
| 12 | 52 | F | 33 | 0 | 0 | 0 | 0 | 0 | 0 | 0 | 0 | 0 | 0 | 0 |
| 13 | 62 | F | 38 | 1 | 1 | 0 | 0 | 1 | 1 | 1 | 1 | 0 | 0 | 0 |
| 14 | 0 | | 76 | 0 | 0 | 0 | 0 | 0 | 0 | 0 | 0 | 0 | 0 | 0 |
| 15 | 85 | M | 58 | 1 | 1 | 0 | 0 | 1 | 0 | 1 | 0 | 1 | 0 | 0 |
| 16 | WD | | 25 | 1 | 0 | 0 | 0 | 0 | 0 | 0 | 0 | 0 | 0 | |
| 17 | 57 | M | 82 | 1 | 1 | 1 | 1 | 1 | 1 | 1 | 0 | 1 | 0 | 0 |
| 18 | 67 | F | 60 | 1 | 0 | 0 | 0 | 0 | 0 | 0 | 0 | 0 | 0 | 0 |
| 19 | 55 | M | 63 | 0 | 0 | 0 | 0 | 0 | 0 | 0 | 0 | 0 | 0 | 0 |
| 20 | 67 | M | 27 | 0 | 0 | 0 | 0 | 0 | 0 | 0 | 0 | 0 | 0 | 0 |
| 21 | 94 | F | 33 | 0 | 0 | 0 | 0 | 0 | 0 | 0 | 0 | 0 | 0 | 0 |
| 22 | WD | | 54 | 0 | 0 | 0 | 0 | 0 | 0 | 0 | 0 | 0 | 0 | |
| 23 | 44 | M | 90 | 0 | 0 | 0 | 0 | 0 | 0 | 0 | 0 | 0 | 0 | 0 |
| 24 | 75 | F | 56 | 0 | 1 | 0 | 1 | 1 | 1 | 0 | 1 | 0 | 0 | 0 |
| 25 | 58 | M | 48 | 1 | 0 | 0 | 0 | 0 | 0 | 0 | 0 | 0 | 0 | 0 |
| 26 | 79 | M | 52 | 0 | 1 | 0 | 1 | 1 | 1 | 1 | 0 | 0 | 0 | 0 |
| 27 | 75 | F | 35 | 1 | 1 | 0 | 0 | 1 | 1 | 1 | 1 | 0 | 0 | 0 |
| 28 | WD | F | | 0 | 0 | 0 | 0 | 0 | 0 | 0 | 0 | 0 | 0 | |
| 29 | WD | M | | 0 | 0 | 0 | 0 | 0 | 0 | 0 | 0 | 0 | 0 | |
| 30 | 75 | F | 56 | 0 | 1 | 0 | 1 | 1 | 1 | 0 | 0 | 0 | 0 | 0 |
| 31 | 92 | F | 57 | 1 | 1 | 1 | 1 | 1 | 1 | 1 | 0 | 1 | 1 | 0 |
| 32 | 87 | M | 63 | 1 | 1 | 0 | 1 | 0 | 1 | 0 | 1 | 1 | 0 | 0 |
| 33 | 77 | M | 43 | 1 | 1 | 0 | 0 | 1 | 1 | 1 | 0 | 1 | 0 | 0 |
| 34 | 79 | F | 40 | 0 | 0 | 0 | 0 | 0 | 0 | 0 | 0 | 0 | 1 | 0 |
| 35 | NT | F | 25 | 0 | 1 | 0 | 0 | 0 | 1 | 1 | 0 | 0 | 1 | 0 |
| 36 | NT | F | 27 | 0 | 1 | 0 | 0 | 0 | 1 | 1 | 0 | 0 | 1 | 0 |
| 37 | NT | M | 52 | 0 | 1 | 0 | 1 | 0 | 1 | 0 | 0 | 0 | 0 | 0 |
| 38 | NT | F | 55 | 1 | 1 | 0 | 1 | 1 | 1 | 1 | 0 | 0 | 0 | 0 |
| | 39 NT | M | 58 | 0 | 1 | 0 | 1 | 1 | 1 | 1 | 1 | 0 | 0 | 0 |
| Mean: | | | | | | | | | | | | | | |
| | 62% | 21 F | 48 | 15 | 21 | 4 | 14 | 16 | 20 | 15 | 8 | 6 | 5 | 0 |
| | | 18 M | | | | 20% | 66% | 76% | 95% | 70% | 38% | 29% | 24% | |

| | | | | | | | | | | | | | | |
|---|---|---|---|---|---|---|---|---|---|---|---|---|---|---|
| ID: Identity Number %: Improvement in symptoms as indicated by the BDI Dur: Indicates if the patient has been stable on GMGB1 for > 6 months Quest: Indicates if the patient completed a qualitative improvement questionnaire BP: Indicates a reduction in blood pressure > 25 mm HG Conc: Indicates report of improvement in cognition and clear thinking Stress: Indicates a report of a reduction in stress levels Mood: Indicates a report of stabilisation of mood Anx: Indicates a report of being less anxious Sleep: Indicates a report of an improved sleep pattern Skin: Indicates a report of an improvement in skin, hair and nail condition Sick: Indicates a report of a reduction in symptoms during cold/flu (sickness behaviour) Effect: Indicates a report of adverse effects other than weight gain | | | | | | | | | | | | | | |

### REFERENCES

Anderson et al., Glucosamine effects in humans: a review of effects on glucose metabolism, side effects, safety considerations and efficacy. Food and Chemical Toxicology 43:187-201 (2005).
Baud and Karin, Signal transduction by tumour necrosis factor and its relatives. Trends Cell Biol. 11:372-77 (2001).
Bonizzi and Karin, The two NFκB activation pathways and their role in innate and adaptive immunity. Trends Immunol. 25:280-88 (2004).
Burstein and Duckett, Dying for NFκB? Control of cell death by transcriptional regulation of the apoptotic machinery. Curr. Opin. Cell Biol. 15:732-37 (2003).
Clark et al., The roles of TNF in brain dysfunction and disease. Pharmacol Ther. 128:519-48 (2010).
D'Acquisto et al., Inhibition of nuclear factor kappa B (NFκB): an emerging theme in anti-inflammatory therapies. Molecular Interventions 2:22-35 (2002).
Dejardin et al., The lymphotoxinβ receptor induces different patterns of gene expression via two NFκB pathways. Immunity 17:525-35 (2002).
Ghosh et al., NFκB and Rel proteins: evolutionarily conserved mediators of immune responses. Annu. Rev. Immunol. 16:225-60 (1998).
Ghosh and Karin, Missing pieces in the NFκB puzzle. Cell 109 (Suppl.):S81-96 (2002).
Greten et al., IKKβ links inflammation and tumorigenesis in a mouse model of colitis associated cancer. Cell 118:285-96 (2004).
Horváth et al., Toxicity of methylsulfonylmethane in rats. Food Chem. Toxicol. 40:1459-62 (2002).
Lawrence et al., IKKα limits macrophage NFκB activation and contributes to the resolution of inflammation. Nature 434:1138-43 (2005).
Lin et al., NFkB functions as both a pro-apoptotic and anti-apoptotic regulatory factor within a single cell type. Cell Death Differ. 6:570-82 (1999).
Playfair and Chain, Immunology at a glance, seventh addition, Blackwells Publishing (2001).
Raison et al., Cytokines sing the blues: inflammation and the pathogenesis of depression. Trends. Immunol. 27:24-31 (2006).
Xiao et al., NFκB inducing kinase regulates the processing of NFκB2 p100. Mol. Cell 7:401-09 (2001).

## Claims

1. A pharmaceutical composition for use in the treatment of non-infectious diseases, wherein the composition comprises :
(a) Methylsulfonylmethane or a pharmaceutically acceptable salt or solvate thereof;
(b) Glucosamine, a glucosamine derivative or a pharmaceutically acceptable salt or solvate thereof, wherein the glucosamine derivate is selected from glucosamine sulphate, N-acetyl glucosamine and quaternised amino glucosamine;
(c) L-glycine or a pharmaceutically acceptable salt or solvate thereof;
(d) Vitamin B₁₂, a derivative thereof or a pharmaceutically acceptable salt or solvate thereof, wherein the vitamin B₁₂ derivate is selected from cobalamin, methyl cobalamin, and hydroxycobalamin;
and wherein the non-infectious disease is selected from the group consisting of a mood disorder, an anxiety disorder, an attention deficit disorder, dementia and stress.

2. A pharmaceutical composition for use in the treatment of non-infectious diseases according to Claim 1 comprising:
(a) Methylsulfonylmethane;
(b) Glucosamine;
(c) L-glycine;
(d) Vitamin B₁₂.

3. A pharmaceutical composition for use in the treatment of non-infectious diseases according to Claim 1 or Claim 2, wherein the combination further comprises one or more additional active agents selected from a norepinephrine reuptake inhibitor (NRI), a selective serotonin reuptake inhibitor (SSRI), a tricyclic antidepressant, and a monoamine oxidase inhibitor.

4. A pharmaceutical composition for use according to Claim 3, wherein the additional active agent is an NRI selected from atomoxetine, reboxetine, viloxazine, maprotiline, bupropion, radafaxine, and combinations thereof.

5. A pharmaceutical composition for use according to Claim 3, wherein the additional active agent is an SSRI selected from fluoxetine, paroxetine, citalopram, escitalopram, fluvoxamine, sertraline, and combinations thereof.

6. A pharmaceutical composition for use according to Claim 3, wherein the additional active agent is a tricyclic antidepressant selected from amitriptyline, amoxapine, butriptyline, clomipramine, desipramine, dibenzepin, dosulepin, doxepin, imipramine, lofepramine, nortriptyline, protriptyline, trimipramine, and combinations thereof.

7. A pharmaceutical composition for use according to Claim 3, wherein the additional active agent is a monamine oxidase inhibitor selected from isocarboxazid, moclobemide, phenelzine, tranylcypromine, selegiline, nialamide, iproniazid, iproclozide, toloxatone, harmala, brofaromine, benmoxin, 5-methoxy-N,N-dimethyltryptamine, 5-methoxy-alpha-methyltryptamine, and combinations thereof.

8. A pharmaceutical composition for use according to any of Claims 1 to 7, further comprising a pharmaceutically acceptable carrier, diluent or excipient.

9. A pharmaceutical composition for use according to Claim 8, wherein the composition comprises 500-6000mg methylsulfonylmethane, 500-6000mg glucosamine, 500-2000mg L-glycine, and 0.02-2mg vitamin B₁₂.

10. A pharmaceutical composition for use according to any of Claims 1 to 9, wherein the mood disorder is depression; the anxiety disorder is selected from panic disorder, obsessive-compulsive disorder, post-traumatic stress disorder, social phobia, specific phobias and generalised anxiety disorder; and the attention deficit disorder is ADHD.

11. A pharmaceutical composition for use according to any of Claims 1 to 10, wherein the treatment of the non-infectious disease results in improved cognitive performance in a subject.

## Patentansprüche

1. Pharmazeutische Zusammensetzung zur Verwendung bei der Behandlung von nichtinfektiösen Erkrankungen, worin die Zusammensetzung Folgendes umfasst:
(a) Methylsulfonylmethan oder ein pharmazeutisch annehmbares Salz oder Solvat davon;
(b) Glucosamin, ein Glucosaminderivat oder ein pharmazeutisch annehmbares Salz oder Solvat davon, worin das Glucosaminderivat ausgewählt ist aus Glucosaminsulfat, N-Acetylglucosamin und quaternisiertem Aminoglucosamin;
(c) L-Glycin oder ein pharmazeutisch annehmbares Salz oder Solvat davon;
(d) Vitamin B₁₂, ein Derivat davon oder ein pharmazeutisch annehmbares Salz oder Solvat davon, worin das Vitamin B₁₂-Derivat ausgewählt ist aus Cobalamin, Methylcobalamin und Hydroxycobalamin;
und worin die nichtinfektiöse Erkrankung ausgewählt ist aus der Gruppe bestehend aus einer Gemütsstörung, einer Angststörung, einer Aufmerksamkeitsdefizitstörung, Demenz und Stress.

2. Pharmazeutische Zusammensetzung zur Verwendung bei der Behandlung von nichtinfektiösen Erkrankungen nach Anspruch 1 umfassend:
(a) Methylsulfonylmethan;
(b) Glucosamin;
(c) L-Glycin;
(d) Vitamin B₁₂.

3. Pharmazeutische Zusammensetzung zur Verwendung bei der Behandlung von nichtinfektiösen Erkrankungen nach Anspruch 1 oder Anspruch 2, worin die Kombination ferner einen oder mehrere zusätzliche Wirkstoffe umfasst, die ausgewählt sind aus einem Norepinephrin-Wiederaufnahme-Hemmer (NRI), einem selektiven Serotonin-Wiederaufnahme-Hemmer (SSRI), einem trizyklischen Antidepressivum und einem Monoaminoxidase-Hemmer.

4. Pharmazeutische Zusammensetzung zur Verwendung
nach Anspruch 3, worin der zusätzliche Wirkstoff ein NRI ist, der ausgewählt ist aus Atomoxetin, Reboxetin, Viloxazin, Maprotilin, Bupropion, Radafaxin und Kombinationen davon.

5. Pharmazeutische Zusammensetzung zur Verwendung
nach Anspruch 3, worin der zusätzliche Wirkstoff ein SSRI ist, der ausgewählt ist aus Fluoxetin, Paroxetin, Citalopram, Escitalopram, Fluvoxamin, Sertralin und Kombinationen davon.

6. Pharmazeutische Zusammensetzung zur Verwendung
nach Anspruch 3, worin der zusätzliche Wirkstoff ein trizyklisches Antidepressivum ist, das ausgewählt ist aus Amitriptylin, Amoxapin, Butriptylin, Clomipramin, Desipramin, Dibenzepin, Dosulepin, Doxepin, Imipramin, Lofepramin, Nortriptylin, Protriptylin, Trimipramin und Kombinationen davon.

7. Pharmazeutische Zusammensetzung zur Verwendung
nach Anspruch 3, worin der zusätzliche Wirkstoff ein Monamineoxidase-Hemmer ist, der ausgewählt ist aus Isocarboxazid, Moclobemid, Phenelzin, Tranylcypromin, Selegilin, Nialamid, Iproniazid, Iproclozid, Toloxaton, Harmala, Brofaromin, Benmoxin, 5-Methoxy-N,N-dimethyltryptamin, 5-Methoxy-alpha-methyltryptamin und Kombinationen davon.

8. Pharmazeutische Zusammensetzung zur Verwendung
nach einem der Ansprüche 1 bis 7, ferner umfassend einen pharmazeutisch annehmbaren Träger, Verdünner oder Exzipienten.

9. Pharmazeutische Zusammensetzung zur Verwendung
nach Anspruch 8, worin die Zusammensetzung 500-6000 mg Methylsulfonylmethan, 500-6000 mg Glucosamin, 500-2000 mg L-Glycin und 0,02-2 mg Vitamin B₁₂ umfasst.

10. Pharmazeutische Zusammensetzung zur Verwendung
nach einem der Ansprüche 1 bis 9, worin die Gemütsstörung Depression ist; die Angststörung ausgewählt ist aus Panikstörung, Zwangsstörung, posttraumatischer Belastungsstörung, sozialer Phobie, spezifischen Phobien und generalisierter Angststörung; und die Aufmerksamkeitsdefizitstörung ADHS ist.

11. Pharmazeutische Zusammensetzung zur Verwendung
nach einem der Ansprüche 1 bis 10, worin die Behandlung der nichtinfektiösen Erkrankung in verbesserter kognitiver Leistung in einem Subjekt resultiert.

## Revendications

1. Une composition pharmaceutique destinée à être utilisée dans le traitement de maladies non infectieuses, dans laquelle la composition comprend :
(a) du méthylsulfonylméthane ou un sel ou un solvate de ce dernier pharmaceutiquement acceptable ;
(b) de la glucosamine, un dérivé de la glucosamine ou un sel ou un solvate de ce dernier pharmaceutiquement acceptable, dans laquelle le dérivé de la glucosamine est sélectionné parmi un sulfate de glucosamine, une N-acétyle glucosamine et une aminoglucosamine quaternisée ;
(c) de la L-glycine ou un sel ou un solvate de ce dernier pharmaceutiquement acceptable;
(d) de la vitamine B₁₂, un dérivé de cette dernière ou un sel ou un solvate de ce dernier pharmaceutiquement acceptable, dans laquelle le dérivé de la vitamine B₁₂ est sélectionné parmi la cobalamine, la cobalamine de méthyle et l'hydroxycobalamine;
et dans laquelle la maladie non infectieuse est sélectionnée dans le groupe constitué par un trouble de l'humeur, un trouble de l'anxiété, un trouble du déficit de l'attention, une démence et un stress.

2. Une composition pharmaceutique destinée à être utilisée dans le traitement de maladies non infectieuses selon la revendication 1 comprenant :
(a) du méthylsulfonylméthane;
(b) du glucosamine ;
(c) de la L-glycine ;
(d) de la vitamine B₁₂.

3. Une composition pharmaceutique destinée à être utilisée dans le traitement de maladies non infectieuses selon la revendication 1 ou la revendication 2, dans laquelle la combinaison comprend en outre un ou plusieurs agents actifs additionnels sélectionnés parmi un inhibiteur de la recapture de la norapinéphrine (IRN), un inhibiteur de la recapture de la sérotonine sélective (IRSS), un antidépresseur tricyclique et un inhibiteur de la monoamine oxydase.

4. Une composition pharmaceutique destinée à être utilisée selon la revendication 3, dans laquelle l'agent actif additionnel est un IRN sélectionné parmi l'atomoxétine, la réboxétine, la viloxazine, la maprotiline, le bupropion, la radafaxine, et des combinaisons de ces derniers.

5. Une composition pharmaceutique destinée à être utilisée selon la revendication 3, dans laquelle l'agent actif additionnel est un IRSS sélectionné parmi la fluoxétine, la paroxétine, le citalopram, l'escitalopram, la fluvoxamine, la sertraline, et des combinaisons de ces derniers.

6. Une composition pharmaceutique destinée à être utilisée selon la revendication 3, dans laquelle l'agent actif additionnel est un antidépresseur tricyclique sélectionné parmi l'amitriptyline, l'amoxapine, la butriptyline, la clomipramine, la désipramine, la dibenzépine, la dosulépine, la doxépine, l'imipramine, la lofépramine, la nortriptyline, la protriptyline, la trimipramine, et des combinaisons de ces dernières.

7. Une composition pharmaceutique destinée à être utilisée selon la revendication 3, dans laquelle l'agent actif additionnel est un inhibiteur de la monoamine oxydase sélectionné parmi l'isocarboxazide, la moclobémide, la phénelzine, la tranylcypromine, la sélégiline, la nialamide, l'iproniazide, l'iproclozide, la toloxatone, l'harmala, la brofaromine, la benmoxine, la 5-méthoxy-N,N-diméthyltryptamine, la 5-méthoxy-alpha-méthyltryptamine, et des combinaisons de ces dernières.

8. Une composition pharmaceutique destinée à être utilisée selon l'une quelconque des revendications 1 à 7, comprenant en outre un porteur, un diluant ou un excipient pharmaceutiquement acceptable.

9. Une composition pharmaceutique destinée à être utilisée selon la revendication 8, dans laquelle la composition comprend 500 à 6000 mg de méthylsulfonylméthane, 500 à 6000 mg de glucosamine, 500 à 2000 mg de L-glycine et 0,02 à 2 mg de vitamine B₁₂.

10. Une composition pharmaceutique destinée à être utilisée selon l'une quelconque des revendications 1 à 9, dans laquelle le trouble de l'humeur est la dépression ; le trouble de l'anxiété est sélectionné parmi un trouble panique, un trouble obsessionnel compulsif, un trouble du stress post traumatique, une phobie sociale, des phobies spécifiques et un trouble de l'anxiété généralisé ; et le trouble du déficit de l'attention est le TDAH.

11. Une composition pharmaceutique destinée à être utilisée selon l'une quelconque des revendications 1 à 10, dans laquelle le traitement de la maladie non infectieuse résulte en une meilleure performance cognitive chez un sujet.
